# EUROPEAN PATENT APPLICATION

(11) **EP 3 992 280 A1**
(43) Date of publication of application: **04.05.2022**
(21) Application number: 20205010.0
(22) Date of filing: 30.10.2020
(51) Int. Cl.: C12N 5/079, C12N 5/071

(54) **METHOD FOR THE GENERATION OF NEUROSPHERES**

(71) Applicant: Kugelmeiers Ltd., 8703 Erlenbach (CH)
(72) Inventor: GOPALAKRISHAN, Jay, 40591 Düsseldorf (DE)
(74) Representative: Stolmár & Partner Patentanwälte PartG mbB

(57) **Abstract**

The present invention relates to a method for producing a plurality of homogeneous neurospheres, said method comprising: providing pluripotent cells; seeding said pluripotent cells into a cell culture device comprising at least one compartment comprising a plurality of microwells; and culturing said pluripotent cells in said cell culture device under conditions that allow the cells to form neurospheres.

## Description

### Field of the invention

The invention relates to methods for the generation of neurospheres and brain organoids as well as brain organoids obtained by these methods. In addition, the invention relates to methods for the production of brain proteins using the brain organoids.

### Background

Research on stem cell derived human 3D brain organoids, also known as mini-brains, have received great attention in the last decade, due to the organoids' enormous potential in modelling brain related developmental disorders.

Brain organoids are capable of self-assembly and form an organized architecture, composed of progenitor, neuronal and glial cell types (Jo, J., Xiao, Y. et al. (2016). Midbrain-like organoids from human pluripotent stem cells contain functional dopaminergic and neuromelanin-producing neurons. Cell Stem Cell 19, 248-257. Kadoshima, T., Sakaguchi, H. et al. (2013). Self-organization of axial polarity, inside-out layer pattern, and species-specific progenitor dynamics in human ES cell-derived neocortex. Proc. Natl. Acad. Sci. USA 110, 20284-20289.2013. Lancaster, M. A., Renner, M. et al. (2013). Cerebral organoids model human brain development and microcephaly. Nature 501, 373-379.). Unlike conventional two-dimensional (2D) cell cultures, brain organoids recapitulate the human brain not only at the cellular level, but also in terms of general tissue structure and developmental trajectory, which is essential to study the brain's physiology and pathology.

Already, self-assembling 3D human brain organoids have been used to accurately model several neurodevelopmental disorders, which has never been possible with 2D cultures in the past (Sutcliffe, M., and Lancaster, M.A. (2019). A Simple Method of Generating 3D Brain Organoids Using Standard Laboratory Equipment. Methods Mol Biol 1576, 1-12.). Therefore, organoid technology could be an important tool to model diseases such as microcephaly, dwarfism, Alzheimer's and even some of the severe form of cancers, e.g. glioblastoma (Gabriel, E., Ramani, A. et al. (2017). Recent Zika Virus Isolates Induce Premature Differentiation of Neural Progenitors in Human Brain Organoids. Cell Stem Cell 20, 397-406 e395. Wang, 2018. Linkous, A., Balamatsias, D. et al. (2019). Modeling Patient-Derived Glioblastoma with Cerebral Organoids. Cell Rep 26, 3203-3211 e3205.).

However, although several groups have been actively working on strategies to optimize production, brain organoids are still notoriously difficult to generate in the lab, in particular in large quantities. The existing methods are labor intensive and time consuming, requiring weeks and often months until finished organoids are obtained. In addition, the generated organoids often show a high degree of heterogeneity in terms of morphology and size, even within a single batch. This could adversely affect their analysis, since large differences in size are likely have an influence on the physiology of these mini-brains (Wang, H. (2018). Modeling Neurological Diseases With Human Brain Organoids. Front Synaptic Neurosci 10, 15.).

In addition, a number of research groups generate organoids from embryoid bodies (EBs), which basically have the capability to generate all the three germ layers. Differentiation of these EBs into the neural lineage generates brain organoids that might additionally harbor cells from other lineages, too, which in turn could affect organoid functionality.

Gabriel et al. 2017 overcame the problem associated with EBs by directly differentiating human induced pluripotent stem cells (hiPSCs) to neuroectoderm, thus avoiding contamination with other lineages (Lancaster, M.A., and Knoblich, J.A. (2014). Generation of cerebral organoids from human pluripotent stem cells. Nat Protoc 9, 2329-2340.). However, this method still allows to generate only a small number of brain organoids at a time.

### Objective problem to be solved

There is therefore a need in the art for a method that allows the generation of a plurality of homogenous brain organoids with similar properties and characteristics.

### Brief description of the figures

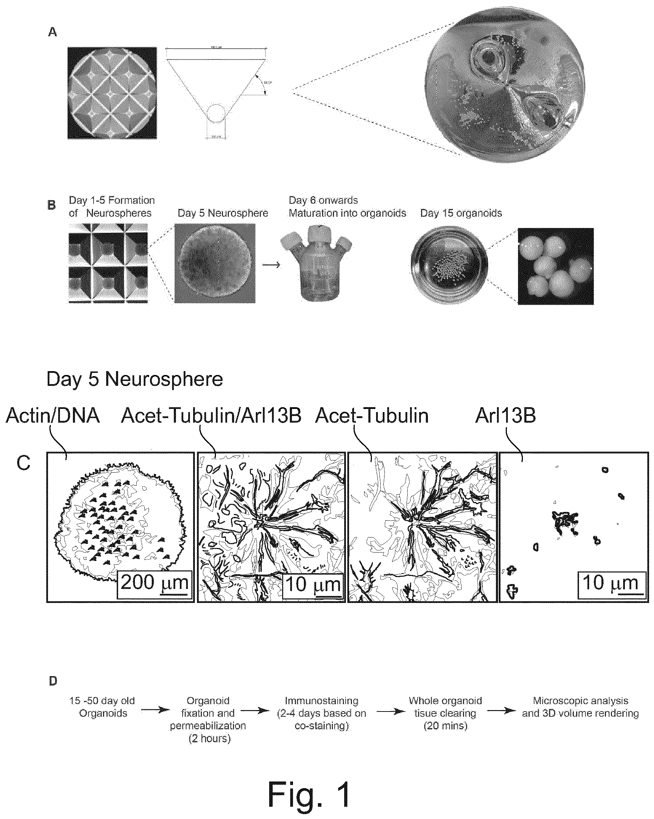

**Figure 1** shows the generation of human 3D brain organoids using the Hi-Q approach A: Micro-patterned plate to generate large number of organoids. B: Method depicting the differentiation of hiPSCs in micro-patterned plates to 3D neurospheres followed by maturation into organoids in spinner flask. C: Preliminary characterization of the 3D neurospheres shows ventricular zones (marked by Actin, green), with primary cilium (marked by Arl13B, red) arranged in the luminal side of the neural rosettes. D: Brief overview of processing and analysis of brain organoids.

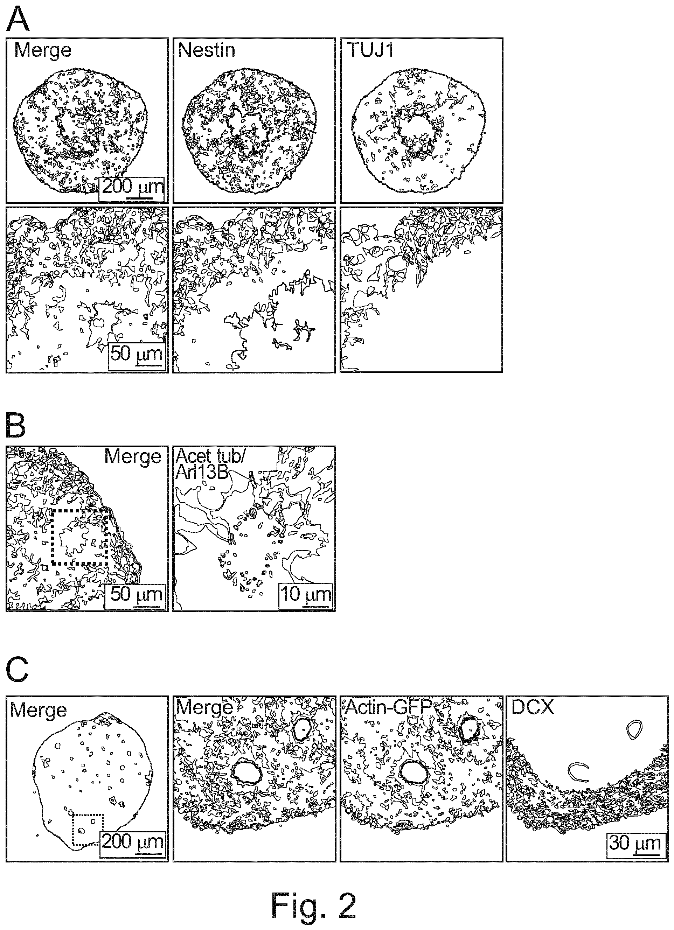

**Figure 2** shows that brain organoids according to the invention are positive for stem cell and neuronal markers. A: Neural stem cell marker (Nestin, green) and Neuron marker (TUJ1, red) shown in tissue cleared whole brain organoid mount. B: Acet-Tub (green) and primary cilium marker Arl13B (red). The cilia seem to be present at the luminal side of the ventricular zone, which is indicative of polarity. C: Organoids stained with Actin (green) and neuronal marker (DCX, red).

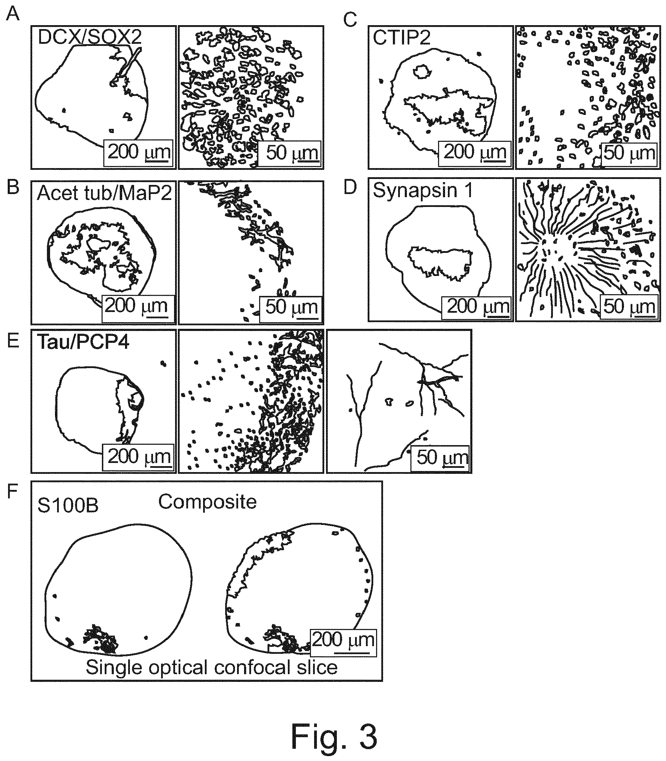

**Figure 3** shows that day 50 Hi-Q organoids exhibit markers for maturation. A: Neural stem cell marker (SOX2, red) shows the presence of VZs. Left panel shows tissue cleared and stained whole organoid. Right panel shows few stacks of a single VZ. B: Microtubule marker (acetylated tubulin, green), co-stained with a matured neuronal marker (Map2, red). C: Post mitotic neuronal marker (CTIP2, red). D: Marker for neuronal Phosphoproteins, that bind to synaptic vesicles (Synapsin 1, red). E: Microtubule associated protein (Tau, green), co-stained with synaptic plasticity marker, also known as purkinje cell protein 4 (PCP4, red). F: Glial marker (SB100, green) shows maturation of organoids.

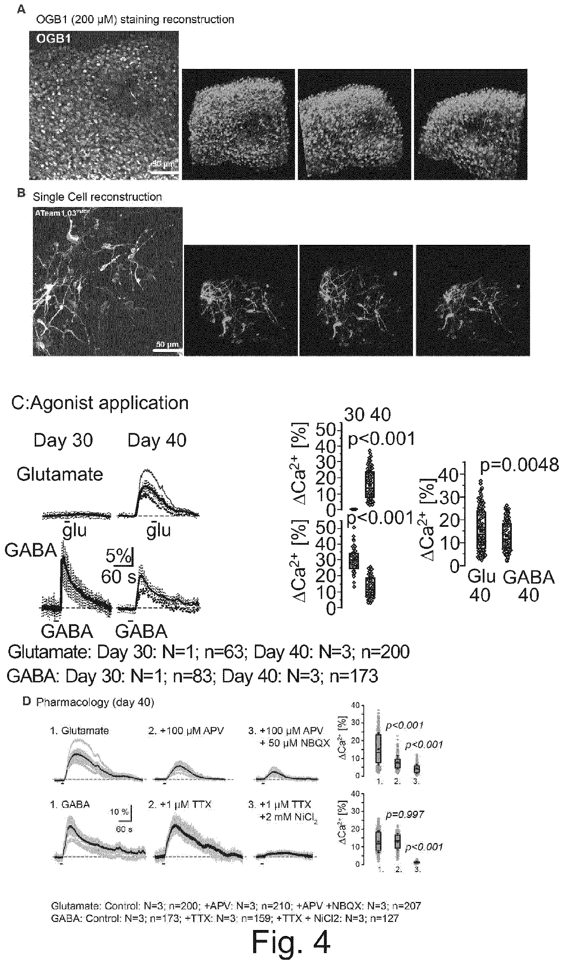

**Figure 4** shows that the generated brain organoids are electrically active. A: Calcium imaging using calcium indicator dye Oregon Green BAPTA 1. B: Epression of ATP-sensor ATeam1.03YEMK under synapsin promoter. C: Day 30 organoids show no response to glutamate but only respond to GABA. D: Day 40 organoids respond to both glutamate and GABA.

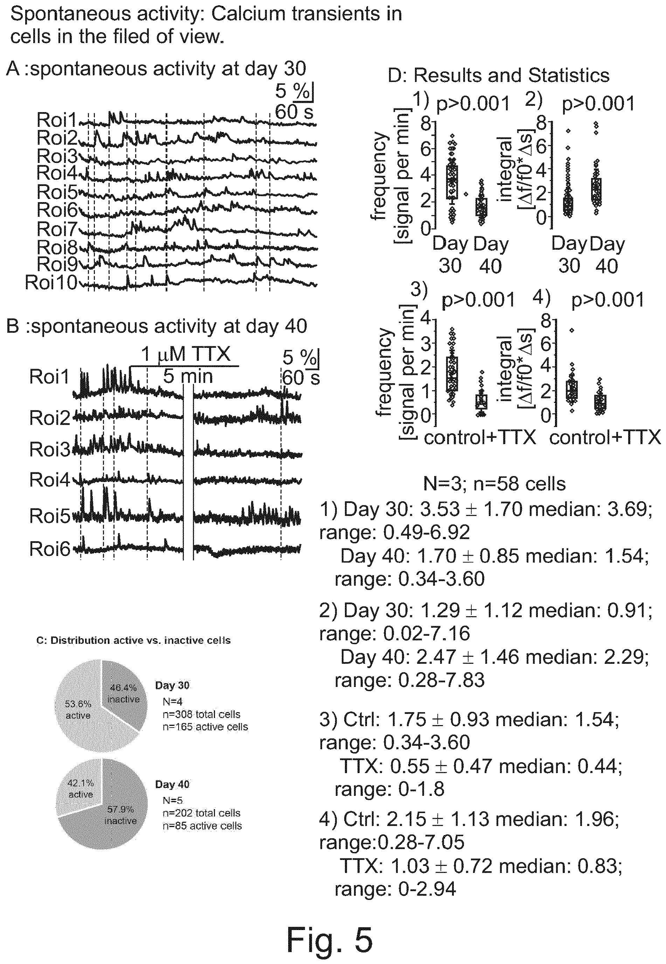

**Figure 5** shows spontaneous calcium currents and inhibition thereof by TTX. A: Calcium imaging was performed to analyze the neuronal activity in both developing and mature organoids by measuring spontaneous calcium currents are seen at Day 30 with an average frequency of 3.5 signals per minute. B: Day 40 organoids with larger amplitude and kinetics probably due to increased neurons, astrocytes and maturing networks. C: Pie chart showing distribution of active vs inactive cells in Day 30 organoids compared to day 40 organoids. D: Addition of TTX (a potent sodium channel blocker) is seen to reduce the action potentials in the cells of organoids indicating that the cells indeed generate electrical currents.

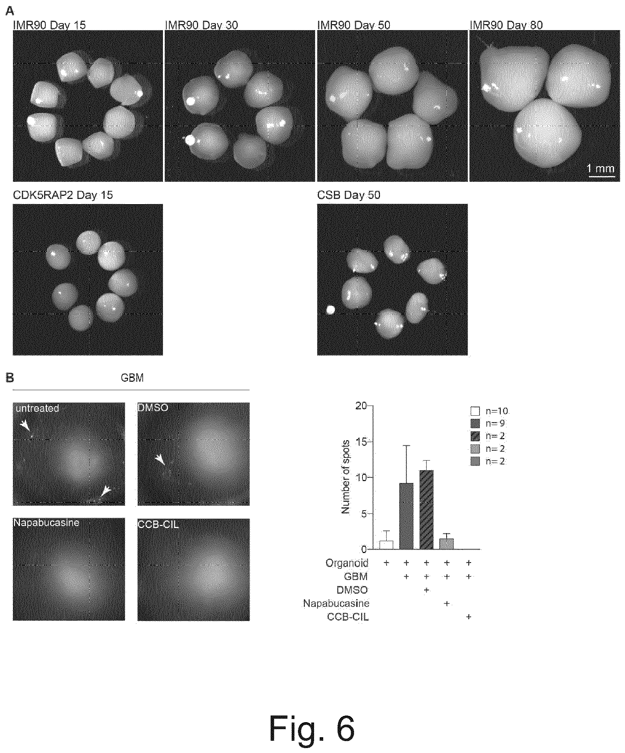

**Figure 6** shows that the approach according to the invention holds promise in disease modelling and medium throughput screening. A: Hi-Q wildtype brain organoids (IMR90) show increase in size spanning from day 15 to day 80. The organoids have similar size within the same timepoint. CDK5RAP2 and CSB organoids have mutations resulting in microcephaly in patients, which is recapitulated by the Hi-Q organoids.

### Summary of the invention

In one aspect, the invention relates to a method for producing a plurality of homogeneous neurospheres, said method comprising:
- providing pluripotent cells;
- seeding said pluripotent cells into a cell culture device comprising at least one compartment comprising a plurality of microwells; and
- culturing said pluripotent cells in said cell culture device under conditions that allow the cells to form neurospheres.

In a second aspect, the invention relates to a method for producing a plurality of homogeneous brain organoids, said method comprising:
- providing pluripotent cells;
- seeding said pluripotent cells into a cell culture device comprising at least one compartment comprising a plurality of microwells;
- culturing said pluripotent cells in said cell culture device under conditions that allow the cells to form neurospheres;
- transferring the formed neurospheres into 3D culture dishes; and
- culturing said neurospheres in said 3D culture dishes under conditions that allow said neurospheres to develop into brain organoids.

In a third aspect the invention relates to brain organoids comprising microglial cells.

In a fourth aspect, the invention pertains to methods for the producing human brain proteins, wherein the human brain proteins are produced by the brain organoids according to the invention.

In a fifth aspect, the invention relates to a method for drug screening using the neurospheres and brain organoids generated by the methods of the invention.

### Detailed description of the invention

In a first aspect, the invention relates to a method for producing a plurality of homogeneous neurospheres, said method comprising:
- providing pluripotent cells;
- seeding said pluripotent cells into a cell culture device comprising at least one compartment comprising a plurality of microwells; and
- culturing said pluripotent cells in said cell culture device under conditions that allow the cells to form neurospheres.

In a second aspect, the invention relates to a method for producing a plurality of homogeneous brain organoids, said method comprising:
- providing pluripotent cells;
- seeding said pluripotent cells into a cell culture device comprising at least one compartment comprising a plurality of microwells;
- culturing said pluripotent cells in said cell culture device under conditions that allow the cells to form neurospheres;
- transferring the formed neurospheres into 3D culture dishes; and
- culturing said neurospheres in said 3D culture dishes under conditions that allow said neurospheres to develop into brain organoids.

Neurospheres are herein defined as free-floating 3D cell clusters comprising neural precursor cells. Neurospheres can be used to develop and generate brain organoids, which are herein defined as self-assembled 3D cell aggregates with cell types and a cytoarchitecture resembling human brain tissue. Put differently, neurospheres can, under the right culture conditions, mature into brain organoids. Brain organoids are also referred to as neural organoids, cerebral organoids or mini-brains.

The methods of the invention are useful for generating a plurality of homogenous neurospheres and brain organoids with minimal effort. The approach has been termed Hi-Q approach and is further described in Fig. 1.

Conventional methods for the generation of brain organoids are usually carried out in a 96 well plate. Therefore, in one round, a maximum of 96 organoids can be obtained from each plate. In contrast thereto, the invention allows the production of several hundred up to several thousand neurospheres and brain organoids from a single cell culture device comprising a plurality of microwells. In one embodiment, up to 10 000 neurospheres may be generated from one cell culture device.

The generation of brain organoids in large, homogenous batches greatly promotes their application for research and diagnostics. For example, large batches are useful for high throughput screening methods for toxicology studies and drug testing. The method of the invention also allows to perform experiments in technical and biological replicates using brain organoids from the same batch, thus ensuring reproducibility.

According to the invention, the plurality of neurospheres or brain organoids obtained by the methods described herein are characterized in that they form a homogenous population, wherein essentially all neurospheres or brain organoids have a similar diameter, shape, anatomy, cytoarchitecture, functionality and cell diversity.

The methods of the invention are also advantageous in that variability between different batches is minimized due to standardized culture conditions.

In a preferred embodiment, the obtained neurospheres and brain organoids all have a similar size. Preferably, the neurospheres have a diameter of 150 micrometer to 500 micrometer, preferably 300 micrometer to 500 micrometer. The diameter of the brain organoids varies depending on the culture duration. When transferred to the 3D culture dish, they have a diameter of about 600 micrometer to 800 micrometer. At day 30, they have a diameter of about 1.0 mm to 1.5 mm. At day 50, they have a diameter of about 1.5 mm to 2 mm.

It is herein defined that the neurospheres have a similar diameter when the standard deviation as measured over the plurality of neurospheres is less than 20%, preferably less than 10%, most preferably less than 5 %. Likewise, it is defined that the brain organoids have a similar diameter when the standard deviation as measured over the plurality of brain organoids is less than 15%, more preferably less than 10%.

In another aspect, the neurospheres and brain organoids obtained by the methods of the invention all have a mostly spherical shape. A mostly spherical shape is herein defined as meaning that the diameter of the neurospheres and brain organoids is essentially the same when measured through different axes of the neurosphere or brain organoid.

In another aspect, the neurospheres and brain organoids according to the invention all show a similar cytoarchitecture, i.e. different cell types are positioned in a similar fashion within the neurospheres and brain organoids. For instance, neural stem cells reside within neural rosettes (in neurospheres) and ventricular zones (in brain organoids), whereas neurons extend outwards to occupy the cortical surface.

In another aspect, the neurospheres and brain organoids according to the invention all show a similar cell diversity. In particular, the brain organoids according to the invention comprise neural stem cells, neurons, astrocytes and microglial cells.

Because the neurospheres and brain organoids according to the invention share the same characteristics in terms of size, cytoarchitecture and electrical properties, their functionality is also homogenous within a population. Functionality of the organoids may be defined by their response to pharmacological agents. The organoids according to the invention show presence of sodium and potassium currents which may be activated or inhibited by chemical compounds. For instance, Tetrodotoxin (TTX), a potent neurotoxin known to block sodium channels, also inhibits sodium channels in the brain organoids according to the invention, as analyzed by calcium imaging experiments (see Fig. 6).

The methods of the invention comprise a first step of providing pluripotent cells. In order to generate neurospheres and later brain organoids, it is necessary that the pluripotent cells have at least the capability to develop into neuroectoderm. The pluripotent cells may also be omnipotent cells. However, in a preferred embodiment, the cells do not have the capability to develop in all three germ layers in order to avoid that the resulting brain organoids contain cells from a germ layer other than neuroectoderm.

In one embodiment, the pluripotent cells are human cells, preferably human stem cells. In another embodiment, the pluripotent cells are embryonic stem cells. In a particularly preferred embodiment, the cells are human induced pluripotent stem cells (hiPSCs), i.e. human somatic cells that have been reprogrammed back into a pluripotent state.

The cells used in the methods of the invention may be commercial hiPSCs such as WISCi004-B (Wicell, WI, USA), AICS-0012 (Allen Cell Collection, Coriell Institute, USA), AICS-0016 (Allen Cell Collection, Coriell Institute, USA) and AICS 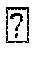 0031 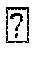 035 (Allen collection, Coriell Institute, USA) or any other iPSCs generated from somatic cells. In one embodiment, patient-derived cells or cells known to carry specific genetic mutations and alterations may be used to generate the iPSCs used in the methods of the invention.

In the next step of the methods according to the invention, the pluripotent cells are seeded into a cell culture device. "Seeding" is herein defined as applying a cell containing solution to a cell culture device in a manner that the cells are evenly distributed over the whole surface of the cell culture device. In a preferred embodiment, the seeding step is performed in a way that each microwell within one compartment receives essentially the same number of cells. In one embodiment, the plate is moved in a figure 8 movement during the seeding step in order to distribute the cells evenly over all microwells.

With methods known in the art, it is necessary to seed the cells manually into each well of a 96 well plate. Because this process cannot be automated, human error is inevitable, which is amplified as the cells multiply, thereby giving rise to differently sized organoids (Sloan, S.A., Andersen, J. et al. (2018). Generation and assembly of human brain region-specific three-dimensional cultures. Nat Protoc 13, 2062-2085.). With the method according to the invention, this error is avoided because it allows to precisely control the number of cells each microwells receives by statistics.

According to the invention, the cells are seeded and cultured in a cell culture device comprising at least one compartment comprising a plurality of microwells. Using such a cell culture device allows to generate a plurality of neurospheres in parallel with minimal effort. In conventional methods, one needs to pipette cells into each well of a 96 well plate and in the next 5 days, all the 96 wells require a partial medium change. This cannot be performed with a multichannel pipette, as it could disrupt the neurospheres (Sloan, S.A., Andersen, J. et al. (2018). Generation and assembly of human brain region-specific three-dimensional cultures. Nat Protoc 13, 2062-2085. Gabriel, E., Ramani, A. et al. (2017). Recent Zika Virus Isolates Induce Premature Differentiation of Neural Progenitors in Human Brain Organoids. Cell Stem Cell 20, 397-406 e395.). Therefore, medium change has to be performed manually which consumes enormous amounts of time. In contrast thereto, the cell culture devices used in the methods of the invention allow to change the medium for all microwells within one compartment in one go, thus greatly reducing the time and effort needed to generate neurospheres.

Because the culture conditions in each of the microwells are essentially the same, the resulting neurospheres and brain organoids will show a high degree of homogeneity in terms of anatomy, cytoarchitecture, functionality and cell diversity.

In one embodiment, in each of the cell culture device's compartments, the plurality of microwells is arranged in a way that the compartment bottom is completely covered in microwells and the compartment bottom is essentially free of flat surfaces. Such an arrangement prevents cells from attaching to surfaces outside the microwells during the seeding step. Put differently, each cell is forced into a microwell. This prevents the uncontrolled development of individual cells outside of the microwells.

In another preferred embodiment, each microwell of the cell culture device has essentially the same volume. This ensures that the growth conditions in each microwell are essentially the same, thus further promoting homogeneity of the generated neurospheres and brain organoids.

It is preferred that the cell culture device used in the methods of the invention has the same footprint as standard multi-well cell culture plates, i.e. a length of about 127 mm, a width of about 85 mm and a height of about 13 to 14 mm. Since labs are used to handling plates in this size and many automatization tools are also designed for cell culture devices having these dimensions, manipulation is greatly facilitated when using such a footprint.

The cell culture device may comprise exactly 1, 2, 3, 4, 5, 6, 8, 10, 12, 18, 24, 48, 96, 384, 1536, 3456 or 9600 compartments. In a preferred embodiment, the cell culture device comprises 24 compartments. A smaller number of compartments requires less manipulation during seeding and culturing. On the other hand, the bigger the surface of one compartment is, the more difficult it is to evenly distribute the cells over the whole surface and thus ensure an equal number of cells in each microwell. 24 compartments have been found to be easy to handle while guaranteeing that all microwells receive essentially the same number of cells during seeding.

The microwells of the cell culture device are dimensioned so to accommodate neurospheres for several days of culture. This is advantageous when using the neurospheres for drug testing. In one embodiment, the microwells have an opening of about 1 mm x 1 mm and a tip diameter of about 180 micrometer. In another embodiment, the microwells have a volume greater than 800 micrometer to 900 micrometer and/or are able to accommodate neurospheres up to day 10 after seeding.

Each compartment of the cell culture device comprises a plurality of microwells. For example, a compartment may comprise between 4 and 10000 microwells.

In one embodiment, a cell culture device such as described in EP 20175312.6 is used in the methods according to the invention. In these cell culture devices, the compartment bottom is completely covered in microwells, with no space between the microwells and no flat surfaces outside the microwells. In addition, all of the microwells have essentially the same volume. These cell culture devices are therefore particularly advantageous when one wants to obtain a plurality of neurospheres or brain organoids showing a high degree of homogeneity.

In one embodiment, the microwells of the cell culture device have a pyramid shape, with the pyramid extending into the bottom of the cell culture device. The pyramids further have a rounded tip and rounded edges between the side walls of the pyramid. Such a geometry without any sharp edges provides a natural growth environment, prevents the formation of unnaturally developing neurospheres and promotes the formation of rounded organoids with a uniform size. The geometry is further described in EP 20175312.6.

According to the methods of the invention, the cells are cultured in the cell culture device under conditions that allow the cells to form neurospheres. Any culture conditions known in the art to mediate the directed differentiation into neuroectoderm and the formation of neurospheres may be applied. In a preferred embodiment, the cells are subjected to directed differentiation conditions, preferably using a serum-free medium comprising 1:1 DMEM/F12 and Neural Basal medium, supplemented with N-2 supplement (1:200), B-27 supplement w/o vitamin A (1:100), 0.05 mM MEM non-essential amino acids, L-glutamine (1:100), insulin (1.6g/L), 0.05 mM β-mercaptoethanol, 5 µM SB431542 and 0.5 µM dorsomorphin.

In the methods according to the invention, the cells are preferably cultured at 37°C with 5% CO₂.

When generating brain organoids according the methods of the invention, the neurospheres are transferred from the cell culture device into a 3D culture dish. In a preferred embodiment, neurospheres are transferred after only a few days of growth in the cell culture device, in particular 4 to 7 days after seeding.

For subsequent culture, any 3D culture dish known in the art may be used. In one embodiment, spinner flasks, bioreactors or orbital shakers are used for further culture. In a preferred embodiment, spinner flaks are used because they are particularly suitable for homogenously distributing the medium and providing maximum perfusion of nutrients into the organoids. In another preferred embodiment, a 3D culture dish with a clinostat supporting simulated microgravity, i.e., a condition which simulates a property of near weightlessness, is used, which greatly enhances cell proliferation.

The 3D culture dishes may be uncoated or (pre-)coated. In a preferred embodiment, the culture dishes are pre-coated, in particular with a non-toxic solution that can be easily washed off prior to use. In one embodiment, the 3D culture dishes are rinsed with Anti-Adherence Rinsing Solution (Catalog #07010, Stemcell Technologies) in order to prevent adhesion of the neurospheres to the walls of the dish.

In a preferred embodiment, the method for the generation of brain organoids comprises detaching the neurospheres from the microwells and then transferring them into a cell strainer. The cell strainer is then inverted, and the organoids are released into a dish using organoid medium. Once all the neurospheres are released, they are transferred into the organoid flaks containing organoid medium. The flaks are then incubated at 37°C and this is marked as Day 0.

In a particularly preferred embodiment, the organoid medium used for cultivation of the brain organoids comprises a serum-free medium composition comprising 1:1 DMEM/F12 and Neural Basal medium, supplemented with N2 supplement (1:200), B-27 supplement w/o vitamin A (1:100), 0.05 mM MEM non-essential amino acids, L-glutamine (1:100), insulin (1.6g/L), 0.05 mM β-mercaptoethanol, 5 µM SB431542 and 0.5 µM Dorsomorphin.

Previously, organoid production involved embryoid body formation, matrigel embedding, neuroepithelial formation and finally transfer into flasks for organoid generation (Karzbrun, E., and Reiner, O. (2019). Brain Organoids - A Bottom-Up Approach for Studying Human Neurodevelopment. Bioengineering (Basel) 6.). In contrast thereto, in one embodiment of the methods of invention, the pluripotent cells do not form embryoid bodies. This further reduces the probability of the brain organoids comprising cells form other germ layers.

Markers for maturity such as glial markers or matured neurons often appear only in brain organoids that are more than 100 days old. The long cultivation time results in large organoids having a size of 3 to 4 mm. Such large brain organoids need to be sectioned for staining, leading to loss of information during the sectioning process.

Using the methods of the invention, it is possible to obtain mature brain organoids within 50 days. The shortened culture time is achieved by growing the neurospheres in the microwell plates for only four days and then directly transferring them to 3D culture dishes for subsequent brain organoid formation.

The methods of the invention reduce the time necessary to obtain mature brain organoids from about 100 days to about 50 days. Because of the shorter cultivation time, the obtained brain organoids are smaller with a diameter of 1 to 1.5 mm and can therefore be cleaned and stained without the need to be sectioned.

Maturity of the brain organoids may be shown by the presence of maturity markers such as Map-2, Synapsin 1 and S100 Beta or by detection of electrophysiological activity.

In one embodiment, the method can be used to generate patient specific brain organoids, which may be useful for providing personalized medicine. In these cases, the pluripotent cells used in step a) are patient specific cells.

In a third aspect, the invention relates to brain organoids comprising microglial cells. Microglial cells are primary immune cells and the resident macrophages of the central nervous system. They can be identified by commonly used antibodies such as anti-lbal (Abcam, United Kingdom), anti-TMM119 (Abcam, United Kingdom), anti-CD45 (Cell signaling technology, USA) and CD11b (Abcam, United Kingdom). Previously described brain organoids do not contain microglial cells; rather, microglial cells had to be cultured separately and then added to mature organoids (Song L, Yuan X, Jones Z, et al. (2019) Functionalization of Brain Region-specific Spheroids with Isogenic Microglia-like Cells. Sci Rep. 2019;9(1):11055.). In contrast thereto, the brain organoids according to the invention comprise this important cell type, making them ideal models to study immune responses and neuroprotection of the brain.

In one embodiment, the brain organoids according to the invention show reduced stress levels compared to brain organoids known from the art. "Reduced stress levels" are herein defined as either a reduction of the levels of stress related genes such as PGK1, ARCN1 and GORASP2 or reducedstress-induced necrosis in the center of the organoids as measured via detection of apoptosis, which can be measured, e.g., using TUNEL (terminal deoxynucleotidyl transferase dUTP nick end labeling) staining.

In another embodiment, the brain organoids according to the invention show electrically active glutaminergic and GABAergic neurons, indicating the maturation process and functionality of the brain organoids, which is also important with regard to using the brain organoids for testing novel drugs.

The person skilled in the art knows how the presence and type of electrically active cells can be detected within brain organoids. For example, calcium imaging using Oregon Green Bapta-1 shows the activity of sodium and potassium channels in different neurons, which may be either activated or blocked when pharmacological agonists or antagonists are administered to the brain organoids.

In yet another embodiment, the brain organoids according to the invention are positive for neural stem cell markers, in particular Synapsin-1, GAD67, Tau, MAP2, CTIP, alpha-Synuclein and VGlut-1, differentiated early neuronal markers, in particular PAX6, SOX2, and Nestin, and/or late neuronal markers, in particular DCX and TUJ1. The person skilled in the art is well aware how detection of markers can be performed. Common techniques include enzyme-linked immunosorbent assay (ELISA) or another immunoassays.

In a preferred embodiment, the brain organoids express at least one neural stem cell marker, one differentiated early neuronal marker and one late neuronal marker. Brain organoids that comprise neural stem cells which differentiate into neuronal stem cells resemble actual human brain tissue.

The brain organoids according to the invention can be frozen and re-cultured with a viability of at least 90%. Viability is herein the ratio of brain organoids that do not disintegrate upon re-culture. In addition, about 90% of the cells within each brain organoid are alive after thawing and re-culture.

Brain organoids showing the characteristics mentioned above can be obtained by the methods according to the invention.

The brain organoids of the invention may be used similarly to how brain organoids have been used in the art. For example, they may be used to model neuronal connectivity or neurodevelopmental disorders. They may also be used to conduct toxicology studies or drug testing. Because of their homogeneity in size, in particular diameter, the brain organoids of the invention are suitable for automated study of their physiology and morphology.

The brain organoids according to the invention are also useful in the treatment or diagnosis of certain diseases and disorders, in particular neurodevelopmental diseases, neurodegenerative diseases, infectious brain diseases, brain cancer, brain tumors, stroke or brain diseases linked to autoimmune conditions. They may also be used in methods of diagnosis and treatment of these diseases and disorders.

In a fourth aspect, the invention pertains to methods for the producing human brain proteins, wherein the human brain proteins are produced by the brain organoids according to the invention. For the brain organoids to produce human brain proteins, the pluripotent cells used to generate the brain organoids may be human cells or may be cells stably expressing human brain proteins. Human brain proteins include insulin, tubulin and disease relevant proteins such as Tau (MAPT).

The skilled person is aware how organoids can be used to produce proteins of interest and how these proteins can be harvested. In one embodiment, pluripotent stem cells overexpressing the protein of interest are used to generate the brain organoids. Regarding the purification, it has been described how tubulin has been purified from porcine brain (Gell C., Friel C.T., et al. (2011) Purification of tubulin from porcine brain. Methods Mol Biol. 2011;777:15-28). A similar approach may be carried out using brain organoids according to the invention.

Because the brain organoids according to the invention closely resemble actual brains, the brain proteins produced by these brain organoids also closely resemble naturally occurring brain proteins, making them suitable for the administration to humans and the use as therapeutic proteins.

In a fifth aspect, the invention relates to a method for drug screening using the neurospheres and brain organoids generated by the methods of the invention. Herein, the neurospheres and brain organoids are used to test the efficacy of different drugs or drug candidates. In one embodiment, the neurospheres and brain organoids are patient specific neurospheres or brain organoids, thus allowing to screen drugs that will be specifically effective for the patient in question (personalized medicine). In another embodiment, the neurospheres and brain organoids are model neurospheres or brain organoids representative for the diseases and disorders mentioned above, so that the method can be used for testing the efficacy of a prospective drug candidate for these diseases and disorders.

In the methods for drug screening according to the invention, the neurospheres and brain organoids are generated using the cell culture device described above. Once the neurospheres are generated, the drug or drug candidate can be directly added to the cell culture device.

In one embodiment, each compartment of the cell culture device comprises only a small number of microwells, e.g., up to 2, 4, 6, 8 or 10 microwells. This is helpful in case that only a small number of pluripotent cells is available, e.g. in case of patient derived pluripotent cells. At the same time, the cell culture device comprises a plurality of compartments, so that many different drugs can be tested using a single cell culture device.

Because the neurospheres and brain organoids generated by the methods of the invention are homogeneous in terms of size, cell diversity and cytoarchitecture, they represent standardized models for testing novel drugs or treatment regimens.

In a preferred embodiment, the method can be used to screen for drugs useful in the treatment of COVID-19 or glioblastoma.

### Examples

### Example 1 - hiPSC generation and maintenance

Four hiPSc lines were tested for the methods described herein. Commercial lines included WISCi004-B (Wicell, WI, USA) and AICS-0012 (Allen Cell Collection, Coriell Institute, USA). The two patient-derived hiPSCs include CDK5RAP2 line (which has a mutation in the centrosomal protein CDK5RAP2) and CSBAS548 line (mutation in the CSB gene). The patient derived lines were obtained from our collaborators. All the cell lines were cultured in a feeder free condition in a serum free medium, mTeSR1 (Stemcell Technologies) on plates coated with Matrigel (Corning). The medium in these dishes were changed once in 3 days and the cells were split 1:2 using an enzyme free method employing ReLSR (Stemcell Technologies) as per the manufacturer's protocol.

### Example 2 - Brain organoid medium composition

Organoids generated with the above mentioned hiPSCs were grown in a serum-free media as described (Gabriel et al., 2017). In brief, the medium comprises of a mixture of 1:1 DMEM/F12 and Neural Basal medium, supplemented with N2 (1:200), B27 w/o Vitamin A (1:100), 0.05mM MEM non-essential amino acids, L-glutamine (1:100), Pen-strep (100 µg/ml each), Insulin (1.6g/L), 0.05 mM β-Mercaptoethanol (Life Technologies), and supplemented with 5 µM SB431542 (Selleckchem, USA) and 0.5 µM Dorsomorphin (Sigma-Aldrich, USA).

### Example 3 - Preparation of organoid flasks

The organoid flasks were prepared 24 hours prior to the start of the experiment. The flasks (Figure 1A and B) were washed with autoclaved double distilled water and autoclaved at 121°C for 15 mins. The autoclaved bottles were allowed to dry under the cell culture hood. Once dried, the flaks and their pendals were coated with a rinsing solution (Stemcell Technologies) for 10 minutes. The solution was removed and the flask and pendals were allowed to dry. Once dried, freshly prepared cold organoid medium containing 0.1% Matrigel (Corning) was added to the flasks. The flasks were then incubated at 37°C for 24 hrs.

### Example 4 - Preparation of microwell plates

1x PBS was added to one well of a Sphericalplate 5D microwell plate containing 185 microwells (Kugelmeiers AG, Switzerland). The plate was centrifuged at 1000 x g for 2-3 minutes to get rid of air bubbles. The PBS was aspirated and 0.5 ml of Neural Induction medium (Stemcell Technologies) was then added to the well and incubated at 37°C till the cells were prepared.

### Example 5 - Generation of brain organoids

hiPScs (at 80% confluency) were dissociated into single cells by treatment with Accutase (Sigma-Aldrich) at 37°C for 5 mins. The cells were then centrifuged at 700 x g for 5 mins and resuspended in 1 ml of Neural induction medium (NIM) (Stemcell Technologies), supplemented with 10 µM ROCK inhibitor, which is a key component to inhibit apoptosis in cells. The cells were counted using a haemocytometer and 1.8 x 10⁶ cells were suspended in 0.5 ml of NIM medium, supplemented with 10 µM ROCK inhibitor. This was then added to the plate containing 0.5 ml of NIM to reach a final volume of 1 ml in each compartment of the microwell plate. The plate was moved in the shape of 8 to evenly distribute the cells. Thus, each microwell received nearly 10,000 cells. The plate was incubated at 37°C.

For the next 5 days, we carried out partial medium change replenishing 0.5 ml of the NIM. During this time, the hiPSCs formed interconnection which gave a 3D appearance also known as neurospheres (Figure 1B). After 5 days of partial medium change, the neurospheres were detached from the microwells by pipetting them with DMEM/F12 medium. These neurospheres were transferred with a cut 1 ml pipette tip into a cell strainer (40µm, Corning). This procedure was repeated until all the neurospheres were detached. The cell strainer was then inverted, and the neurospheres were released into a 10 cm petridish using the organoid medium described. Once all the neurospheres were released, they were transferred into the pre-treated organoid flaks containing the organoid medium. The flaks were then incubated at 37°C. This was marked as Day 0.

### Example 6 - Organoid fixation, permeabilization, antigen retrieval and blocking

Whole brain organoids were fixed for 1 hr using 3.7% Paraformaldehyde at 37°C. The organoids were then washed with 1x PBS containing 30 mM glycine (2-3 times), permeabilized with 1x PBS containing 0.5% Triton X 100, 0.1% Tween 20 and further blocked with 1x PBS containing 0.5% Fish Gelatin for 1 hour at room temperature (RT). For Sox2 staining, the organoids were treated with sodium citrate buffer (10 mM Sodium citrate, 0.05% Tween 20, pH 6.0) at 90 °C for 45 mins after the permeabilization step to carry our antigen retrieval. The organoids were then blocked with 1x PBS containing 0.5% Fish Gelatin for 1 hour at room temperature (RT).

### Example 7 - Immunofluorescent staining

The organoids were fixed and blocked as described above. The organoids were then moved to 2.0 ml Eppendorf tubes for immunostaining. Mouse anti-Nestin (1:100, Novus Biologicals), mouse anti-SOX2 (1:50, abcam), rabbit anti-Arl13b (1:100, Proteintech), rabbit anti-Doublecortin (1:100, Synaptic Systems), rabbit anti-MAP2 (1:200, Proteintech), rabbit anti-synapsin-1 (1:200, Cell Signalling), Rabbit anti-S100 Beta (1:100, Abcam), Rabbit anti-TUJ1(1:400, Sigma - Aldrich), Mouse anti-acetylated tubulin (1:400, Sigma-Aldrich), Mouse anti-Tau (1:100, DSHB), Rat anti-CTIP2 (1:300, Abcam), Rabbit anti-PCP4 (1:100, Proteintech) were used.

For secondary antibodies, we used Alexa Fluor Dyes conjugated either with goat/donkey anti-mouse, anti-rabbit or anti-rat (1:1000, molecular probes, Thermo Fisher, USA). DAPI 1 µg/ml (Sigma Aldrich, USA) was used to stain nuclei.

### Example 8 - Whole organoid tissue clearing and mounting

The organoids were cleared after immunostaining using increasing concentrations of ethanol (50%, 70% and 100%). The organoids were treated with different percentages of ethanol for approximately 5-6 minutes at RT with constant mixing. After treatment with 100% ethanol, the alcohol was removed, and the organoids are allowed to dry till all the ethanol evaporated. The organoids were then cleared by adding 100 µl of ethyl cinnamate (Sigma Aldrich) for 10 minutes. Once cleared, the organoids were transferred with a brush to µ-slide angiogenesis chamber (Cat. No. 81506, Sigma Aldrich), which could readily be imaged.

### Example 9 - Cyto-architectural analysis

The organoids are positive for pluripotency and neural stem cell markers and show apical basal polarity, which is indicated by presence of cilium at the apical size of the ventricular zone (Kashyap et al., 2009) (Figure. 2A and 2B). As these mini-brains mature, we see the presence of differentiated neurons especially at the cortical surface. (Figure. 2A and 2C). Immunostaining of organoids generated using the Hi-Q approach has a distinct advantage over the conventional methods, since we have overcome the need for tissue sectioning (Figure. 1D). Conventional methods generate organoids which are larger in size (approximately 3-4mm) and therefore require to be sectioned. However, with the Hi-Q approach, we are able to generate organoids which range from 0.5mm to 1mm in diameter, which can be easily immuno-stained. In summary, although the Hi-Q method generates smaller sized organoids, these organoids seem to be well patterned at the early stages, which is indeed an important milestone for generating higher number of organoids with less variability in their size and shape. This has been repeatedly demonstrated by us in several rounds or organoid generation.

We have also been able to appreciate the mature markers in our Day 50 organoids. This provides evidence that our organoids are biologically maturing in comparison to the early day organoids (Figure 3). Day 50 Hi-Q organoids exhibit mature neuronal markers such as Map2, PCP4 and synaptic vesicle marker such as synapsin-1 (Figure 3B and E). In addition, these organoids

### Example 10 - Electrophysiological studies in brain organoids

Apart from checking their cytoarchitecture, we also prove that these organoids are physiologically active. We performed a number of electrophysiological experiments, which also include inhibitors, to show that these organoids do possess action potentials in their neurons. In addition, maturation of these organoids has also been demonstrated in these experiments.

In brief, we firstly performed calcium imaging in sections of Hi-Q organoids because, action potentials (which result from the opening of voltage-gated sodium-channels) induce the opening of voltage-gated calcium channels, which then results in influx of calcium from the extracellular space. In these experiments, we detected spontaneous calcium activity in the upper cortical layers, and this in turn indicates the presence of voltage gated sodium channels (Figure 4A and Figure 5). Moreover, we also demonstrated the presence of GABAergic and glutaminergic neurons due to the spike in calcium signals on addition of GABA or glutamate (Figure 4C-D). This was very evident in the older organoids compared to the younger counterparts, which also fits with the maturation process. Signals were additionally blocked by NBQX, which prove the involvement of AMPA receptors. The response to GABA was not altered by TTX but blocked by NiCI2, which provides an evidence that the currents were induced by the opening of voltage-gated calcium channels in response to a depolarization. Therefore, we could successfully demonstrate structural and functional maturation of Hi-Q organoids within a span of 50 days, unlike some conventional methods in the organoid field, where the organoids are grown for several months.

At present, to test the reliability of Hi-Q organoids in modeling neurodevelopmental disorders, we generated 300 microcephaly brain organoids from a patient-derived iPSC line, which could model microcephaly due to a mutation in CDK5RAP2, a human centrosomal protein (Figure 6A) (Yigit et al., 2015). We also used the organoids to model glioblastoma invasion and were able to successfully perform a medium throughput screening with FDA approved compounds ((Figure 6B). In summary, we anticipate the use of Hi-Q approach for large-scale drug discovery, automation assays, disease modeling, and bioprocess experiments.

## Claims

1. A method for producing a plurality of homogeneous neurospheres, said method comprising:
• providing pluripotent cells;
• seeding said pluripotent stem cells in a cell culture device comprising a plurality of compartments comprising a plurality of microwells; and
• culturing said pluripotent stem cells in said cell culture device under conditions that allow the cells to form neurospheres.

2. A method for producing a plurality of homogeneous brain organoids, said method comprising:
• providing pluripotent cells;
• seeding said pluripotent cells in a cell culture device comprising a plurality of compartments comprising a plurality of microwells;
• culturing said pluripotent cells in said cell culture device under conditions that allow the cells to form neurospheres;
• transferring the formed neurospheres into 3D culture dishes; and
• culturing said neurospheres in said 3D culture dishes under conditions that allow said neurospheres to develop into brain organoids.

3. The method according to claim 2, wherein the brain organoids develop within less than 50 days.

4. The method according to any of claims 1 to 3, wherein in each compartment, the plurality of microwells is arranged in a way that the compartment bottom is completely covered in microwells and wherein the compartment bottom is essentially free of flat surfaces.

5. The method according to any of claims 1 to 4, wherein each microwell has the same volume.

6. The method according to any of claims 1 to 5, wherein each microwell has a pyramid shape having a rounded tip and rounded edges between the side walls of the pyramid.

7. The method according to any of claims to 1 to 6, wherein during the step of seeding, each microwell within a compartment receives the same number of pluripotent cells.

8. The method according to any of claims 1 to 7, wherein the pluripotent cells are human pluripotent cells, preferably human induced pluripotent stem cells.

9. Brain organoids comprising microglial cells.

10. The brain organoids according to claim 9, wherein the brain organoids show reduced stress levels, e.g. as assessed by the expression level of stress markers such as PGK1, ARCN1 and GORASP2.

11. The brain organoids according to claim 9 or 10, wherein the brain organoids show electrically active glutaminergic and GABAergic neurons and/or are positive for neural stem cell markers, in particular PAX6, SOX2, Nestin; differentiated early neuronal markers, in particular DCX and TUJ1; and/or late neuronal markers, in particular Synapsin-1, GAD67, Tau, MAP2, CTIP, alpha-Synuclein and VGlut-1.

12. The brain organoids according to claim 9 for use in the treatment or diagnosis of neurodevelopmental diseases, neurodegenerative diseases, infectious brain diseases, brain cancer, brain tumors, stroke or brain diseases linked to autoimmune conditions.

13. The brain organoids according to any of claims 9 to 12, wherein the brain organoids can be frozen and re-cultured with a viability of at least 90%.

14. A method for producing human brain proteins, wherein the human brain proteins are produced by the brain organoids according to any of claims 9 to 13.

15. A method for a drug screening, said method comprising using the brain organoids according to any of claims 9 to 13.
